Europäisches Patentamt

(19) European Patent Office

Office européen des brevets

(11) Publication number: 0 112 061
A2

## (12) EUROPEAN PATENT APPLICATION

(21) Application number: 83307034.5

(22) Date of filing: 17.11.83

(51) Int. Cl.³: A 61 K 31/155
//(A61K31/155, 31/70)

(30) Priority: 20.11.82 GB 8233152

(43) Date of publication of application:
27.06.84 Bulletin 84/26

(84) Designated Contracting States:
BE CH DE FR GB IT LI NL SE

(71) Applicant: BEECHAM GROUP PLC
Beecham House Great West Road
Brentford Middlesex(GB)

(72) Inventor: Newsome, Peter Martin
The Limes 20A York Road
Cheam Surrey(GB)

(74) Representative: Cresswell, Thomas Anthony et al,
European Patent Attorney Beecham Pharmaceuticals
Great Burgh Yew Tree Bottom Road
Epsom Surrey KT18 5XQ(GB)

(54) Pharmaceutical or veterinary compositions containing guanoxabenz.

(57) A pharmaceutical or veterinary oral rehydration composition for treating diarrhoea or scours comprises guanoxabenz or an acid addition salt thereof, in aqueous solution also containing at least 0.5% w/v of an actively absorbed monosaccharide, at least 25m$M$ sodium ions and having an osmolarity of less than 500 mOsmolar.

EP 0 112 061 A2

TITLE MODIFI⸗ ⸗
see front page

## COMPOUNDS AND USE

The present invention relates to guanoxabenz and
its use in the treatment of diarrhoea and scours.

European Patent Application No. 25 269 describes
the use in the treatment of diarrhoea and scours of a
class of compounds having $\alpha$-agonist and vasoconstrictor
activity.  Surprisingly guanoxabenz, which has
$\alpha$-agonist and vasoconstrictor activity and falls within
that class of compounds, has particular advantages in
such treatment.

Accordingly the present invention provides, in one
aspect a method for treating diarrhoea and scours,
which method comprises administering an effective,
non-toxic amount of guanoxabenz or an acid addition
salt thereof, to a human or non-human animal in need of
such treatment.

Alternatively the present invention provides a
pharmaceutical or veterinary composition for use in
treating diarrhoea or scours, which composition
comprises guanoxabenz or an acid addition salt thereof
and a pharmaceutically or veterinarily acceptable
carrier therefor.

Guanoxabenz [1-(2,6-dichlorobenzylideneamino)-3-hydroxyguanidine] is a known compound which is disclosed, together with processes for its production, in UK Patents Nos. 1 253 248 and 1 253 249.

Suitable acid addition salts are formed from pharmaceutically or veterinarily acceptable inorganic and organic acids and include the salts of hydrochloric, hydrobromic, hydroiodic, nitric, sulphuric, citric, lactic, maleic, pamoic and tartaric acids.

It is believed that a suitable dose of guanoxabenz, or a salt thereof, for use in the present treatment, would be in the range 0.005 to 100 mg/kg, which dose may be repeated as and when required. Particularly suitable ranges include 0.001 to 10 mg/kg and 0.05 to 10 mg/kg and especially 0.02 to 0.2 and most preferably about 0.05 mg/kg. Nevertheless it will be appreciated that the dose used in practice will depend on the species to which the dose is administered and on the route of administration.

Pharmaceutical and veterinary compositions of guanoxabenz and acid addition salts thereof (hereinafter referred to as the "drug") will, of course, be adapted for administration to the humans or animals to be treated. Thus, for example, the composition may be a shaped composition, such as a bolus, tablet or capsule. In such cases the pharmaceutically or veterinarily acceptable carrier will be chosen from the usual range of lubricants, dispersants, binders, fillers and the like. When these shaped compositions are for administration to cattle and pigs often they may for instance weigh at least 1 g, on occasions at least 2 g.

For administration to humans, especially children, the drug may suitably be presented as a syrup including suitable colouring and/or flavouring agents. Such syrups are conveniently presented in unit or multi-dose containers.

For veterinary use the composition may also be a dispersion or a solution of the drug in a suitable vehicle for use with an oral doser (this is a well known item of farm equipment, basically comprising a liquid reservoir, a mouthpiece adapted for insertion into animals mouths, and a pump mechanism whereby unit doses can be ejected from the reservoir through the mouthpiece). Conveniently the drug may be administered from an oral doser as an aqueous solution. Alternatively, the vehicle will be an oil or water based cream to ensure homogeneity of the unit doses administered.

The invention, therefore, also provides an oral doser containing a multi-dose of the drug in a veterinarily acceptable vehicle.

The drugs of the invention may also be added to the animal feed or drinking water. Thus the invention also provides animal feed or animal drinking water containing a compound of formula (I). It will be convenient to formulate these animal feed and drinking water compositions with a multi-dose of the drug so that the animal takes in an appropriate quantity of the drug along with its diet. It will also be convenient to present the composition of the invention as a premix for addition to the feed or drinking water.

With human babies or young animals, a particularly useful technique is to blend the drug with the milk provided for them.

The compositions of the invention may also be formulated for injection. In such cases the drug chosen is suitably dissolved in water for injection. Alternatively the drug may be administered in a solution used for parenteral fluid replacement therapy.

Treatment of diarrhoea and scours using the drug may be supplemented by oral rehydration therapy such as those described in U.K. Patent No. 1,581,826 and German Offenlegungsschrift No. 28 54 281, UK Patent Application No. 2 012 163A, US Patent No. 3 898 328, Nalin, D.R. and Cash, R.A., Bull. World Health Org., 43, 361 (1970), French Patent No. 2 467 599, UK Patent No. 1 465 308 and as described in "Secretory Diarrhoea", Ed M. Field, J.S. Fordtran and S,G, Schultz, American Physiological Society, Maryland, 1980 pp 179-185 and Lancet, (1975) pp 79 and 80. Conveniently the drug may be administered with the oral rehydration formulation.

Accordingly the present invention provides, in a particular aspect, a formulation for treating diarrhoea which comprises an effective non-toxic amount of a compound of formula (I) as hereinbefore defined and an oral rehydration composition comprising a pharmacologically acceptable aqueous solution containing at least 0.5% w/v of an actively absorbed monosaccharide, at least 25 mM sodium ions and having an osmolarity less than 500 m Osmolar.

Preferably the oral rehydration composition further comprises actively-absorbed amino acids and electrolytes.

The drug may be presented as a formulation containing one or more components of the oral rehydration composition for admixture with the remaining components.

Alternatively the drug may be provided separately and administered simultaneously or sequentially with the oral rehydration formulation.

Often it will be appropriate to include in the compositions a further medicine such as an antibacterial agent for example an antibiotic such as amoxycillin or neomycin or a sulphonamide such as sulfadoxin, an agent to alter intestinal motility such as loperamide or a material such as pectin.

The amount of drug administered must, of course, be sufficient to bring about the desired effect and will also depend on the body weight of the recipient and the chosen route of administration. Useful dosage units based on such dosage would contain from 0.1 mg to 500 mg of the drug, more suitably 0.2 mg to 500 mg. Of course, it will be appreciated that many preferred compositions of the invention are in multi-dose form as, for the therapy of animals, it is often most desirable to be able rapidly to treat a number of animals. Such multi-dose compositions will contain, by way of example, at least 1 mg of the drug. Depending on the exact nature of the said multi-dose composition, often it will contain at least 50 mg of the drug, and on occasions as much as 5 g. Doses may be administered once or several times daily.

The following Formulations illustrate the invention but are not intended to limit the invention in any way.

As used in the Formulations the term "Drug" refers to guanoxabenz or an acid addition salt thereof, in particular the hydrochloride salt.

## Formulation of the Compounds for Veterinary Administration

## Formulation 1: Bolus

Boluses of the following composition were prepared:-

| Drug | 100 mg |
|---|---|
| Microcrystalline cellulose | 500 mg |
| Corn starch | 250 mg |
| Magnesium stearate | 25 mg |
| Lactose, anhydrous | to 2500 mg |

The ingredients were passed through a 30 mesh stainless steel screen and blended in a suitable blender. The resultant compression mix was compressed directly on a tabletting machine to give tablets each containing 10 mg of the compound of Example 1.

Formulation 2:

Oral Doser 1 mg/g

    1 Kg of the following composition was prepared:-

|  | % by wt. |
|---|---|
| Drug | 0.1 |
| Aluminium stearate | 6.0 |
| Sunflower oil | to 100 |

The aluminium stearate was dispersed with stirring in a portion of the sunflower oil heated to 115°C. The dispersion was added to the rest of the sunflower oil heated to 140°C. The gel was stirred at 130°C for 15 minutes and then allowed to cool without stirring to room temperature. The milled drug was dispersed in the cooled gel base and then passed through a colloid mill to produce a fine, homogenous dispersion. The dispersion was filled into plastic bottles fitted with a dosing pump.

Formulation 3
================

Injection 5 mg/ml
=================

    1 litre of the following composition was prepared:-

|  | % w/v |
|---|---|
| Drug | 0.5 |
| Sodium chloride | 0.5 |
| Water for injections | to 100 |

The drug and sodium chloride were dissolved in the water for injections and the solution was filtered and filled into glass ampoules. The ampoules were sterilised by membrane filtration.

## Formulation 4

### Soluble Powder

1 Kg of the following composition was prepared:-

|  | % by wt. |
|---|---|
| Drug | 0.7 |
| Ascorbic acid | 1 |
| Lactose | to 100 |

The drug and lactose were sieved and mixed together in a suitable blender to give a homogenous powder. The powder was filled into jars. The powder was used at the rate 1 g per gallon of drinking water to medicate pigs.

Formulation 5

Oral Rehydration Formulation

1 kg of the following composition was prepared by mixing together the ingredients in dry powder form:

| | |
|---|---|
| Glycine | 10.3 % |
| Dextrose (anhydrous) | 67.6 |
| Sodium Chloride | 14.3 |
| Potassium Dihydrogen Phosphate | 6.8 |
| Citric Acid | 0.8 |
| Tri-potassium Citrate | 0.2 |
| Drug | 0.003 |

60 g of the composition was then dissolved in 2 litres of water and fed to diarrhoeic calves.

Formulation 6

The following formulation may be prepared by the method set out below:-

| | |
|---|---|
| Drug | 0.1 % w/v |
| Bentone 38 (1) | 1.5 % w/v |
| | (ie 1.5 g/100ml) |
| Propylene Carbonate | 0.6 % w/v |
| Pharmasorb (2) | 10 % w/v |
| Phosphoric Acid (3) | 0.1 % w/v |
| Ampicillin Trihydrate | 6.0 % w/v as free acid |
| Soya-Bean Oil | to 100 % |

(1) Bentone 38 is an amide derivative of bentonite

(2) Pharmasorb is a brand of activated Attapulgite,

(3) The phosphoric acid is present to balance the alkaline pH of the Bentone.

The Bentone was dispersed in the soya-bean oil, and when thoroughly distributed, the propylene carbonate was added with high speed mixing, followed by colloid milling to produce the base. Into this base was first mixed the phosphoric acid, and then the pharmasorb, the penicillin, and the drug and the resultant suspension was then passed through a colloid mill once more.

CLAIMS

1.  A pharmaceutical or veterinary oral rehydration
    composition for treating diarrhoea or scours
    characterised in that the composition contains
    guanoxabenz or a pharmaceutically or veterinarily
    acceptable acid addition salt thereof.

2.  A composition as claimed in claim 1 comprising a
    pharmacologically acceptable aqueous solution
    containing at least 0.5% w/v of an actively
    absorbed monosaccharide, at least 25m$\underline{M}$ sodium ions
    and having an osmolarity less than 500 mOsmolar.

3.  A composition as claimed in claim 2 additionally
    comprising actively absorbed amino acid and
    electrolyte.

4.  A composition as claimed in claim 2 or claim 3
    comprising citric acid or a citrate salt or a
    mixture thereof.

5.  A composition as claimed in claim 4 wherein the
    actively absorbed monosaccharide is glucose, the
    actively absorbed amino acid is glycine, the
    sodium ions are provided by sodium chloride and
    the electrolyte is potassium dihydrogen phosphate.

6.    A composition as claimed in any one of claims 1 to
      5 in unit dosage form containing from 0.1 to 500mg
      of guanoxabenz or a pharmaceutically or
      veterinarily acceptable acid addition salt thereof
      per dosage unit.

7.    Use of a composition as claimed in any one of
      claims 1 to 6 for treating diarrhoea or scours.

8.    A composition as claimed in any one of claims 1 to
      6 for use in treating diarrhoea or scours.

9.    Use of guanoxabenz or a pharmaceutically or
      veterinarily acceptable acid addition salt thereof
      for treating diarrhoea or scours.

10.   A process for producing a composition as claimed
      in any one of claims 1 to 6 comprising bringing
      into association an oral rehydration composition
      and guanoxabenz or a pharmaceutically or
      veterinarily acceptable acid addition salt.